(19) Europäisches Patentamt / European Patent Office / Office européen des brevets

(11) **EP 3 728 557 B1**

(12) **EUROPEAN PATENT SPECIFICATION**

(45) Date of publication and mention
of the grant of the patent:
**25.03.2026 Bulletin 2026/13**

(21) Application number: **18815665.7**

(22) Date of filing: **07.12.2018**

(51) International Patent Classification (IPC):
**C12N 5/00** *(2006.01)*

(52) Cooperative Patent Classification (CPC):
**C12N 5/0018;** C12N 2500/50; C12N 2510/02;
C12N 2511/00

(86) International application number:
**PCT/EP2018/083906**

(87) International publication number:
**WO 2019/121053 (27.06.2019 Gazette 2019/26)**

(54) **A METHOD FOR STABILIZING CELL CULTURE SYSTEMS USING AN AMPHIPHILIC GRAFT COPOLYMER AS CELL CULTURE REAGENT**

VERFAHREN ZUR STABILISIERUNG VON ZELLKULTURSYSTEMEN UNTER VERWENDUNG EINES AMPHIPHILEN PFROPFCOPOLYMERS ALS ZELLKULTURREAGENS

PROCÉDÉ DE STABILISATION DE SYSTÈMES DE CULTURE CELLULAIRE À L'AIDE D'UN COPOLYMÈRE GREFFÉ AMPHIPHILE EN TANT QUE RÉACTIF DE CULTURE CELLULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2017 US 201762608247 P**

(43) Date of publication of application:
**28.10.2020 Bulletin 2020/44**

(73) Proprietor: **BASF SE**
**67056 Ludwigshafen am Rhein (DE)**

(72) Inventors:
• **GUTH, Felicitas**
**67056 Ludwigshafen (DE)**
• **KOLTER, Karl**
**67056 Ludwigshafen (DE)**
• **LANGLEY, Nigel A**
**Tarrytown, NY 10591 (US)**
• **FREIDL, Kerri Gaumer**
**Florham Park, NJ 07932 (US)**
• **BRUEGGEMANN, Kristina**
**67059 Ludwigshafen (DE)**

(74) Representative: **BASF IP Association**
**BASF SE**
**GBI - Z078**
**67056 Ludwigshafen (DE)**

(56) References cited:
**WO-A1-2015/003773      WO-A2-03/084479**

• **WEIWEI HU ET AL: "The potential of hydrodynamic damage to animal cells of industrial relevance: current understanding", CYTOTECHNOLOGY, vol. 63, no. 5, 22 July 2011 (2011-07-22), Dordrecht, pages 445 - 460, XP055545586, ISSN: 0920-9069, DOI: 10.1007/ s10616-011-9368-3**
• **MICHAEL LINN ET AL: "Soluplus as an effective absorption enhancer of poorly soluble drugs in vitro and in vivo", EUROPEAN JOURNAL OF PHARMACEUTICAL SCIENCES, ELSEVIER, AMSTERDAM, NL, vol. 45, no. 3, 14 February 2012 (2012-02-14), pages 336 - 343, XP028440388, ISSN: 0928-0987, [retrieved on 20111208], DOI: 10.1016/J.EJPS.2011.11.025**

- **HAOFAN PENG ET AL: "Mechanism investigation for poloxamer 188 raw material variation in cell culture", BIOTECHNOLOGY PROGRESS, vol. 32, no. 3, 1 May 2016 (2016-05-01), pages 767 - 775, XP055545565, ISSN: 8756-7938, DOI: 10.1002/btpr.2268**
- **SAKAI KENTARO ET AL: "Use of nonionic surfactants for effective supply of phosphatidic acid in serum-free culture of Chinese hamster ovary cells", JOURNAL OF BIOSCIENCE AND BIOENGINEERING, ELSEVIER, AMSTERDAM, NL, vol. 92, no. 3, 1 January 2001 (2001-01-01), pages 256 - 261, XP002255475, ISSN: 1389-1723, DOI: 10.1263/JBB.92.256**

**Description**

The claimed invention relates to a method for stabilizing cell culture systems using an amphiphilic graft copolymer as a stabilizing cell culture reagent, wherein the cell culture reagent is a PEG- PVAc-PVCap graft copolymer. Specifically, the cell culture reagent is used as a shear protectant, particularly in suspension cell culture of human or animal cells.

Genetically engineered insect and mammalian cells (typically Chinese hamster ovary "CHO" cells) are used as host organism in the biotechnological production of monoclonal antibodies. Such cells are very sensitive to shear stress as it typically occurs in sparged bioreactors. To date, Poloxamer 188 is the most effective known shear protectant and it is used in any cell culture medium, in complex media and in chemically defined media, that are sold or prepared for the described purpose. The polymer is effective at a concentration of only 1 g/L (0.1%). Nevertheless, the absence of poloxamer 188 has a tremendous negative impact: Cells are dying and thus the yield of the target protein, often a monclonal antibody, is dramatically reduced (Tharmalingam T, et al., Mol Biotechnol. (2008)).

The use of poloxamer 188 in mammalian cell culture was already described in the late 1980s. It was proposed that a certain hydrophobicity of the polymer is essential for the action as shear protectant. The polymer is believed to interact with the cell membrane and the hydrophobic domain is important for that action. However, polymers with a larger hydrophobic portion or potent surfactants are described to have a detrimental effect (cell damage). This hypothesis has not yet been rejected and seems still valid.

The general problem and methods for testing are for example described in the following references:

Murhammer, D.W. et al.: Sparged animal cell bioreactors: Mechanism of cell damage and Pluronic F- 68 protection. Biotechnology Progress, 1990, 6, 391-397;
Murhammer, D.W. & Goochee, C.F.: Structural features of nonionic polyglycol polymer molecules responsible for the protective effect in sparged animal cell bioreactors. Biotechnology Progress, 1990, 6, 142-148;
Tharmalingam, T.et al.: Pluronic enhances the robustness and reduces the cell attachment of mammalian cells. Molecular Biotechnology, 2008, 39, 167 -177;
Hilton, M.D.: Small-scale liquid fermentations. In: Demain, A.L. and Davies, J.E. (Editors): Manual of Industrial Microbiology and Biotechnology 2nd Edition. American Society of Microbiology, Washington, 1999.

The disadvantage is that even very small deviations in poloxamer 188 quality which cannot be detected by standard analytical techniques, can have a negative impact on industrial scale production. For that reason, several manufacturers developed small scale shake flask cell culture models to qualify batches as cell culture grade (Peng H, et al., Biotechnol. Progress (2014)). Even though such quality control procedures have been established the necessary batch screening might be cumbersome and expensive.

There are not many alternatives to poloxamer 188: Alkylgluco- or alkylmalto- pyranosides were proposed as small molecule alternatives by Chalmers and Hu (Hu W, et al: Biotechnology and Bioengineering 2008; Chalmers J; et al: US 20060840552, priority date: August 28th 2006). Octyl - nonyl- and decyl maltopyranosides were found to be less toxic than the corresponding glucopyranosides, but cell growth was already inhibited at relatively low concentrations (for decyl maltopyranoside at a concentration of 0.38 g/L). (Hu, W.: Biotechnology and Bioengineering, Vol. 101, No. 1, September 1, 2008). Maltopyranosides with branched hydrophobic tails were found to be more suitable in their ability to provide sufficient protection at reasonable concentration while also showing reduced toxicity compared to the straight chain counterparts (Wuu, J. et al.: Evaluating sugar-based detergents as a potential alternative to poloxamer bubble protectant.( Conference on Cell Culture Engineering XV, May 8-13, La Quita Resort & Club, Greater Palm Springs Area, California. USA, 2016; Conference Abstract). This shows that alkylmaltopyranosides can only be used within a narrow concentration range to balance the desired protective effect with undesired impairment of cell growth.

Another problem associated with block polymers of the poloxamer type is the content of aldehydes, such as acetaldehyde or propionic aldehyde.

Such aldehydes can react with functional groups of antibodies such as amine groups and thus have a negative effect on antibody stability or cause hypersensibilities.

Aldehydes, such as formaldehyde, acetaldehyde, propionaldehyde, or glyoxal can react with side chains of amino acids (lysine, histidine and cysteine) and form Schiff Bases. Such reactions can impair the efficacy and the stability of the protein.

Reactive aldehydes can also be generated during the oxidative degradation of unsaturated compounds. Such species are particularly undesired because their presence can lead to the addition of reactive carbonyl functional groups in proteins. Such "protein carbonylation" can trigger further undesired reactions, such as cross linking of proteins (Grimsud, P.A. et al.: Oxidative Stress and Covalent Modification of Protein with Bioactive Aldehydes. Journal of Biological Chemistry (283), 32, pg 21837-21841).

According to US 6448371 aldehyde impurities can be removed by treating a solution of the block copolymers with an acid. This however means that the product has to be undergo an additional treatment step.

WO 2015/003773 A1 relates to dry powder cell culture medium comprising at least one polymer embedded compound.

MICHAEL LINN ET AL: "Soluplus as an effective absorption enhancer of poorly soluble drugs in vitro and in vivo", EUROPEAN JOURNAL OF PHARMACEUTICAL SCIENCES, vol. 45, no. 3, 14 February 2012, pages 336-343, describes Soluplus® as an absorption enhancer.

WO 03/084479 A2 describes a method of large scale virus protection.

HAOFAN PENG ET AL: "Mechanism investigation for poloxamer 188 raw material variation in cell culture", BIOTECHNOLOGY PROGRESS, vol. 32, no. 3, 1 May 2016, pages 767-775 describes a mechanism investigation for poloxamer 188 raw material variation in cell culture.

SAKAI KENTARO ET AL: "Use of nonionic surfactants for effective supply of phosphatidic acid in serum-free culture of Chinese hamster ovary cells", JOURNAL OF BIOSCIENCE AND BIOENGINEERING, vol. 92, no. 3, 1 January 2001, pages 256-261 relates to the use of nonionic surfactants for effective supply of phosphatidic acid in serum-free culture of Chinese hamster ovary cells.

Therefore, the problem to be solved was to find materials useful as stabilizers against reactive impurities and as shear protectants in cell culture or similar applications which allow for avoiding the disadvantages of the prior art materials.

The problem was solved by using amphiphilic PEG- PVAc-PVCap graft copolymers as a stabilizing cell culture supplement . The stabilizer is specifically used as a shear protectant.

The stabilizer is also used for stabilizing the produced antibodies against reactive impurities. Reactive impurities are peroxides or aldehydes. Particularly, reactive impurities are aldehydes.

One embodiment of the invention relates to a method for stabilizing cells in cell culture production, the method comprising culturing a cell line capable of expressing proteins in cell culture media, and supplementing said cell culture media with a graft polymer in which N-vinyl caprolactam and vinyl acetate moieties are grafted on a polyethylene glycol backbone, wherein the graft polymer is a product of i) 30 to 70 wt.-% N-vinyl caprolactam moieties, ii) 15 to 35 wt.-% **vinyl** acetate moieties, and iii) 10 to 30 wt.-% of a polyethylene glycol, with the proviso, that the total i), ii), and iii) equals 100 % b.w., wherein the graft polymer is present in amounts of 0.05 to 5 % by weight, based on the total amount of the cell culture media.

According to a most preferred embodiment the graft polymer is a product of i) 40 to 60 wt.-% N-vinyl caprolactam moieties, ii) 15 to 35 wt.-% vinyl acetate moieties, and iii) 10 to 30 wt.-% of a polyethylene glycol.

According to a particularly preferred embodiment the graft polymer is a product of 57% by weight N-vinylcaprolactam, 30% by weight vinyl acetate and 13% by weight polyethylene glycol PEG 6000.

The graft polymers defined above and used in accordance with the invention are also referred to a PEG- PVAc-PVCap graft copolymers.

The polyethylene glycol moiety can have molecular weights in the range of from 200 to 10.000 g/mole, preferably 400 to 8000 g/mole, more preferably 800 to 7000 g/mol. Particularly preferred polyethylene glycol moieties have molecular weights in the range of from 1000 to 6000 g/mol.

The molecular weights of the graft copolymer can range from 10.000 to 150.000 g/mole, preferably 20.000 to 140.000 g/mole, particularly preferred in the range of from 90.000 to 140.000 g/mol.

The synthesis of such graft copolymers is disclosed in WO 2007/051743.

The graft polymers consist of a hydrophilic polyethylene glycol backbone grafted with random copolymer chains of N-vinyl caprolactam and vinyl acetate.

The particularly preferred PEG-VCap-VAc graft polymer is a product of 13% by weight polyethylene glycol PEG 6000, 57% by weight N-vinylcaprolactam and 30% by weight vinyl acetate with a molecular weight measured by gel permeation chromatography in the range of from 90.000 to 140.000 g/mol, which is commercially available as Soluplus ®, BASF SE.

The PEG- PVAc-PVCap graft copolymers can be used also as a mixture with one or more additional shear protectants. Preferably, such additional shear protectant is poloxamer 188. Other shear protectants to be used in a mixture can be polysorbate-20 or polysorbate-80 or straight or branched chain alkyl-βD-maltosides (such as n-octyl-β-D-maltopyranoside or n-decyl-β-D-maltopyranoside.)

The ratio of PEG- PVAc-PVCap graft copolymers to additional shear protectant can range from 10:1 to 1:10.

However, particularly preferred, only PEG- PVAc-PVCap graft copolymer is used as sole shear protectant to achieve the lowest possible aldehyde contents.

The inventive method is applicable to all kind of human or animal cell lines used in cell culture.

The inventive method using a PEG-VCap-VAc graft polymer as stabilizer and shear protectant is especially important for cells grown in suspension culture. Some cells can naturally live in suspensions culture without adhesion to a surface, others need to be adapted to growing in suspension without adherent conditions.

The cell lines are derived from a variety of human or animal organisms, mammalian or non-mammalian and are often modified by genetic engineering, e.g. Chinese or Syrian hamster, murine (mouse), rat, monkey, e.g. African green monkey, bovine, porcine, ovine, canine, fruit bat, fish, e.g. trout, Chinook salmon or zebra fish, amphibians such as frogs or toads, e.g. south African clawed toad, as well as from insects.

Typical insect species are the fall armyworm (Spodoptera frugiperda), cabbage moth (Mamestra brassicae), cabbage looper (Trichopulsia ni), fruitfly (Drosophila melanogaster), gypsy moth, silk worm, tiger moth. These examples are not meant to be limiting.

The morphological type can be epithelial, endothelial, mesenchymal or embryonal, including stem cells. The origin source can be all kind of tissues such as bone, bone marrow, brain, cervix, colon, kidney and urinary tract, liver, lung, lymph node, mammary gland, muscle, nerve, ovary, pancreas, pituitary, pleura, prostate or skin. The cell lines can be derived from normal tissue or malignoms such as carcinomas, sarcomas, melanoma, blastomas or lymphomas and leukaemias. Included are modified T-cells or antigen-activated T-cells.

Commonly known mammalian cell lines are CHO (Chinese Hamster Ovary) such as CHO-K1, CHO-S, CHO-Dux-B11, HEK (Human Embryonal Kidney) 293, Caco-2 (Colon carcinoma), HeLa (Cervix carcinoma)., MDCK (canine kidney); BHK-21 (Baby Hamster Kidney), HT-1080 (Human fibrosarcoma), Vero (African green monkey kidney)

Mouse myeloma (N50) or mouse embryo (NIH3T3); human hepatoma line (HepG2); NS0 myeloma cells; Human fetal: PER.C6 cell lines, MRC-5 cell lines, RA273, WI-38

A commonly used insect cell line is Sf9 from Spodoptera frugiperda. Another commonly used insect cell line is High Five, aka BTI-TN-5B1-4, from Trichopulsia ni.

Apart from these cell lines a great number of cell lines is known and commercially available. The skilled person can find such information electronically in cell line libraries, data bases or in manufacturer's catalogues.

The cells are grown in specific cell culture media comprising as major components carbon sources such as glucose or glutamine, amino acids as building blocks for proteins, vitamins, balanced salt mixtures for optimum osmolarity and essential metal ions, dyes as pH indicator, and buffer to maintain a balanced pH.

The terms "culture media" or "culture medium" refer to any medium that is capable of supporting growth, maintenance, propagation and or expansion of cells in an artificial in vitro environment outside of a multicellular organism or tissue. Cell culture media may be optimized for a specific cell culture use, for example cell culture production medium that is formulated to promote recombinant protein production.

Undefined, complex media components of animal or plant origin, such as serum, yeast extract, or soy hydrolysates are still widely used and can often not be avoided. Chemically defined media with a better traceability and lot-to-lot consistency are generally preferred and present advantages in process development and regulatory considerations.

Even though the composition is defined and each compound can be specified, chemically defined media (chemically defined basal media and chemically defined feed media) often comprise one hundred or more chemical entities. Improvements aim to avoid complex hydrolysates of unknown composition and to eliminate animal derived components.

The term "culture medium" refers to any medium that is capable of supporting growth, maintenance, propagation and or expansion of cells in an artificial in vitro environment outside for a multicellular organism or tissue. Cell culture media may be optimized for a specific cell culture use, including, for example, cell culture growth medium which is formulated to promote cellular growth or cell culture production medium that is formulated to promote recombinant protein production. Cell culture media may include, in whole or in part, standard cell culture media or modified cell culture media. Modified cell culture media may be derived from standard culture media (also known as basal media) by a person skilled in the art.

Modified or enriched cell culture media can contain additional compounds, nutrients, or non-nutrients, trace elements, growth promotors or other ingredients that increase cell culture performance e.g. FunctionMAX™ Titer Enhancer for CHO cells (Life Technologies).

Suitable standard cell culture media include, but are not limited to Dulbecco's modified Eagle's Medium (DEMEM), Minimal Essential Medium (MEM), alpha MEM, Basal Medium Eagle (BME), Glasgow's Minimal Essential Medium (G-MEM),

Suitable chemically defined basal media are commercially available and include, but are not limited to CD FortiCHO™, CD OptiCHO™ (both Life Technologies), ActiCHO-P (GE Healthcare), Ex-Cell™ CD CHO (Sigma Aldrich), ProCHO™5 (Lonza), Cellvento™ CHO-100 (EMD Millipore),

Suitable chemically defined feed media are commercially available and include, but are not limited to IS CHO Feed-CD (Irvine Scientific), BalanCD™ CHO Feed Medium (1-3 (Irvine Scientific, CHO Feed Bioreactor Supplement (Sigma-Aldrich), CHO CD Efficient Feed TM B nutrient supplement (Life Technologies), ActiCHO Feed A and Feed B (both GE Healthcare), CHO CD EfficientFeed™ A (Life Technologies),

Modified or enriched cell culture media can also contain one or more surfactants (Hossler, P., WO201415190 or Hossler, P.: Improvement of Mammalian Cell Culture Performance Through Surfactant Enabled Concentrated Feed Media. Biotechnol.Prog., 2013, Vol 29, No 4 pg1023-1032) with the aim to improve cell culture performance or maintain concentrated media in solution. PEG- PVAc-PVCap graft copolymer (Soluplus®) is a polymeric solubilizer and can therefore provide the same benefit as the low molecular surfactants that are typically used for this purpose.

Having an amphiphilic structure, PEG- PVAc-PVCap graft copolymer has a detectable critical micelle concentration (7.6 mg/L) which is much lower than what is found for typical small molecule surfactants (Reintjes, T.: BASF Pharma Polymers Solubilizer Compendium, Lampertheim, 2011). In an aqueous solution, PEG-PVAc-PVCap graft copolymers form polymer aggregates that are larger than the micelles formed by typical small molecule surfactants. These properties are similar to those described for poloxamer 188 and due to these properties, the polymer can be well tolerated by the cells.

Shear protectants are typically used in amount of from 0.05 to 5. wt.-% and preferably from 0.5 to 3 wt.-%.

Cell culture processes can occur under a variety of environmental conditions in batch or continuous mode. The cell culture process is developed according to the specific needs of the host cell line and the nature of the target proteins. Typically a cell culture step for the manufacture of monoclonal antibodies in a batch or fed batch process takes approximately 14 days. Typical growth conditions for cell cultures are 33-37 °C, $CO_2$ content 5 %, and 95 % humidity. The conditions and the media feeds are adapted to the needs of the specific cell line and are known to those skilled in the art.

After cell harvesting by centrifugation or filtration, target proteins, such as monoclonal antibodies are purified by a sequence of chromatographic and membrane based operations. The capture step (for antibodies, typically protein A chromatography) is followed by ion exchange and hydrophobic interaction chromatography. A virus inactivating operation, a filtration-based virus-reducing step and a final diafiltration are also an important part.

An overview about recent developments in industrial scale cell culture can be found for example in Gronemeyer, P. et al.: Trends in Upstream and Downstream Process Development for Monoclonal Antibodies. Bioengineering 2014, 1, pp 188-212.

PEG- PVAc-PVCap graft copolymers can easily be removed by purification steps that are typically performed in industrial scale cell culturing.

To demonstrate the efficacy of the amphiphilic graft copolymer small scale cell culture experiments were used to evaluate the performance as shear protectant in suspension culture of cells capable of producing monoclonal antibodies.

The general set-up of such shake flask test methods is known in the art, see references cited above. The tests measure cell viability and growth rates of the cultured cells.

Cell viability and growth rate are calculated as specified in connection with the examples.

In cell cultures with PEG- PVAc-PVCap graft copolymer or mixtures of PEG- PVAc-PVCap graft copolymer with second shear protectant cumulative growth rates and cell viability proved to be in the same range as a standard poloxamer 188.

This was surprising, because PEG- PVAc-PVCap graft copolymer (Soluplus®) was developed to increase the oral bioavailability of poorly soluble actives and is a potent solubilizer. PEG-PVAc-PVCap graft copolymer also has a significantly higher molecular weight than poloxamer 188 (average molecular weight of approximately 8.500 Da) or other substances described for such use.

PEG- PVAc-PVCap graft copolymers are stable and do not contain reactive impurities such as peroxides or aldehydes in a free form or trapped into the polymer as acetals. It does not include unsaturated compounds.

**Examples**

The small- scale cell culture experiment was designed to evaluate the performance of Soluplus and mixtures of Soluplus with poloxamer P188 as shear protectant in suspension culture of Chinese hamster ovary (CHO) cell lines capable of producing monoclonal antibodies. The test below was carried out to identify statistically significant performance differences at a confidence levels of > 95%.

Pluronic® F68, a commercially available poloxamer P188, was used as a reference standard.

The PEG-VCap-VAc graft polymer was a commercially available product obtained from 13% by weight polyethylene glycol PEG 6000, 57% by weight N-vinylcaprolactam and 30% by weight vinyl acetate (Soluplus ®, BASF SE).

Materials & Methods

Cell line:
CHO-S-IgG1
Seeding Density:
$3 \times 10^5$ cells/mL; Cells were passaged without poloxamer 188 in the media for at least 5 passages prior to setting up the experiment.

EP 3 728 557 B1

Volume after Inoculation: 50 mL
Shake flasks: 500 mL, baffled, Nalgene sterile vented shake flasks (Fisher Cat# 10-531) Agitation: 225 rpm
Cell culture Medium: HyClone™ HyCell™ CHO Medium (General Electric Health Care, USA, Cat # SH30933.01) - supplied without shear protectant
Run Duration: 4 days
Incubator: Infors HT Multitron

Shear protectant:
Pluronic® F68 (reference standard)
Commercially available PEG-VCap-VAc graft copolymer: 57% by weight N-vinylcaprolactam, 30% by weight vinyl acetate and 13% by weight polyethylene glycol PEG 6000, MW in the range of 90.000 to 140.000 g/mole; Soluplus®, Fa. BASF SE
Concentration of shear protectant: 0.25 g/L

Analysis
Viable Cell Counts, Total Cell Counts and % Viability: 300 $\mu$L sample were run on a Roche CEDEX automated cell counter using Trypan Blue exclusion.

Cell viability and growth rate were calculated as follows:

$$\text{Total Cell Density (TDC)[1/mL]} = \frac{\text{Number of total cells } [-]}{\text{Sample volume [mL]}}$$

$$\text{Viable Cell Density (VDC)[1/mL]} = \frac{\text{Number of viable cells } [-]}{\text{Sample volume [mL]}}$$

$$\text{Viability [\%]} = 1.00 - \left(\frac{\text{Number of dead cells}}{\text{Total cell count}}\right) \cdot 100$$

$$\text{Specific Growth rate (day}^{-1}) = \left(\ln \frac{\text{Viable cell density (t)}}{\text{Viable cell density (t0)}}\right)$$

Cumulative growth rate: calculated for 3 and 4 days.
Ln(Viable Cell Density Day 3,4/Viable Cell Density Day 0)/3

Statistical analysis: Unpaired Student t-test to determine if differences are significant (>95% confidence)

Results

Table 1

| | AVERAGE: Cumulative Viable Cell Growth Rate (day-1) from Day 0 to Day X | | STANDARD DEVIATION: Cumulative Viable Cell Growth Rate(day-1) | |
|---|---|---|---|---|
| | X=D3 | X=D4 | X=D3 | X=D4 |
| F68 | 0,80 | 1,03 | 0,01 | 0,02 |
| F68/ PEG-VCap-VAc 3:1 | 0,74 | 0,98 | 0,01 | 0,02 |
| F68/ PEG-VCap-VAc 1:3 | 0,82 | 1,04 | 0,05 | 0,05 |
| PEG-VCap-VAc | 0,78 | 1,01 | 0,03 | 0,03 |
| Without shear protectant | - 0.27 | - 0.55 | 0.3 | 0.5 |

7

The results show that Soluplus has a performance as shear protectant comparable to the reference standard.

Aldehyde content

The aldehyde contents of the different samples were tested according to the following method:
The aldehydes were determined by reversed phase HPLC after reaction of the sample with 2,4-dinitrophenylhydrazine as the respective dinitrophenyl hydrazones. For quantification, an external standard was applied using UV detection at 370 nm.

Sample derivatization: 60 mg of polymer (poloxamer 188 or PEG-PVCap-PVAc graft copolymer were weighed (accurate to 0.01 mg) into a 10 mL volumetric flask, dissolved in 1 mL acetonitrile, and derivatized by addition of 1-2 mL reagent solution followed by heating to 60°C for 5 min. After cooling down to ambient temperature, the flask is filled up to the mark with acetonitrile/ water (1:1)

Reagent solution: Approx. 4 g with 2,4-dinitrophenylhydrazine (stabilized with 50 % water) re weighed into a 1 L Erlenmeyer flask. 800 mL water and 200 mL concentrated hydrochloric acid were added. The mixture is stirred until it is clear.

Stationary phase: Symmetry Shield RP 18 - 5$\mu$m, Waters (2,1 mm diameter, stainless steel) Calibration solutions: 20 mg of aldehyde dinitrophenylhydrazones were weighed, accurate to 0.01 mg, and dissolved in acetonitrile. Dilutions were adjusted in such a way that the concentration of hydrazine is within the following ranges:

| | |
|---|---|
| Formaldehyde derivative | 0.0021 - 0.43 mg/10 mL injection solution |
| Acetaldehyde derivative | 0.0024 - 0.47 mg/10 mL injection solution |
| Propionic aldehyde derivative | Approx. 0.0024 - 0.47 mg/10 mL injection solution |

Mobile phase: water (A) / acetonitrile (B) gradient:

| t/min | A [%] | B [%] |
|---|---|---|
| 0 | 60 | 40 |
| 25 | 30 | 70 |
| 35 | 30 | 70 |
| 36 | 60 | 40 |
| 45 | 60 | 40 |

Flow: 0.4 mL/min
Injection volume: 5 $\mu$L
Temperature: 45°C
Detection: UV/VIS, lambda = 370 nm

The total aldehyde contents listed in the tables for the starting materials were calculated based on the hydrazone derivatives content.

Results:

Table 2

| Sample | Formaldehyde [ppm] | Acetaldehyde [ppm] | Propionic aldehyde [ppm] |
|---|---|---|---|
| Poloxamer 188 | < 20 | 163 | 257 |
| PEG-VCap-VAc | < 20 | <5 | <5 |

**Claims**

**1.** A method for stabilizing cells in cell culture production, the method comprising culturing a cell line capable of expressing proteins in cell culture media, and supplementing said cell culture media with a graft polymer in which N-vinyl caprolactam and vinyl acetate moieties are grafted on a polyethylene glycol backbone, wherein the graft polymer is a product of i) 30 to 70 wt.-% N-vinyl caprolactam moieties, ii) 15 to 35 wt.-% vinyl acetate moieties, and iii) 10 to 30 wt.-% of a polyethylene glycol, with the proviso, that the total i), ii), and iii) equals 100 % b.w., wherein the graft polymer is present in amounts of 0.05 to 5 % by weight, based on the total amount of the cell culture media.

2. The method according to Claims 1, wherein the graft polymer is a product of i) 40 to 60 wt.-% N-vinyl caprolactam moieties, ii) 15 to 35 wt.-% vinyl acetate moieties, and iii) 10 to 30 wt.-% of a polyethylene glycol.

3. The method according to Claim 1 or 2, wherein the PEG-VCap-VAc graft polymer is a product of 57% by weight N-vinylcaprolactam, 30% by weight vinyl acetate and 13% by weight polyethylene glycol PEG 6000.

4. The method according to any of Claims 1 to 3, wherein the graft polymer is present in amounts of 0.5 to 3 % by weight, based on the total amount of the media.

5. The method according to any of Claims 1 to 4, wherein the graft polymer is used in combination with a second stabilizer.

6. The method according to Claim 5, wherein the second stabilizer is a shear protectant.

**Patentansprüche**

1. Verfahren zur Stabilisierung von Zellen bei der Zellkulturproduktion, wobei das Verfahren das Kultivieren einer Zelllinie, die Proteine in Zellkulturmedium exprimieren kann, und das Ergänzen des Zellkulturmediums mit einem Pfropfpolymer, in dem N-Vinylcaprolactam- und Vinylacetat-Einheiten auf eine Polyethylenglykolhauptkette aufgepfropft sind, umfasst, wobei es sich bei dem Pfropfpolymer um ein Produkt aus i) 30 bis 70 Gew.-% N-Vinylcaprolactam-Einheiten, ii) 15 bis 35 Gew.-% Vinylacetat-Einheiten und iii) 10 bis 30 Gew.-% eines Polyethylenglykols handelt, mit der Maßgabe, dass die Summe von i), ii) und iii) gleich 100 Gew.-% ist, wobei das Pfropfpolymer in Mengen von 0,05 bis 5 Gew.-%, bezogen auf die Gesamtmenge des Zellkulturmediums, vorliegt.

2. Verfahren nach Anspruch 1, wobei es sich bei dem Pfropfpolymer um ein Produkt aus i) 40 bis 60 Gew.-% N-Vinylcaprolactam-Einheiten, ii) 15 bis 35 Gew.-% Vinylacetat-Einheiten und iii) 10 bis 30 Gew.-% eines Polyethylenglykols handelt.

3. Verfahren nach Anspruch 1 oder 2, wobei es sich bei dem PEG-VCap-VAc-Pfropfpolymer um ein Produkt aus 57 Gew.-% N-Vinylcaprolactam, 30 Gew.-% Vinylacetat und 13 Gew.-% Polyethylenglykol PEG 6000 handelt.

4. Verfahren nach einem der Ansprüche 1 bis 3, wobei das Pfropfpolymer in Mengen von 0,5 bis 3 Gew.-%, bezogen auf die Gesamtmenge des Mediums, vorliegt.

5. Verfahren nach einem der Ansprüche 1 bis 4, wobei das Pfropfpolymer in Kombination mit einem zweiten Stabilisator verwendet wird.

6. Verfahren nach Anspruch 5, wobei es sich bei dem zweiten Stabilisator um ein Scherschutzmittel handelt.

**Revendications**

1. Procédé pour stabiliser des cellules dans la production de cultures cellulaires, le procédé comprenant la culture d'une lignée cellulaire capable d'exprimer des protéines dans des milieux de culture cellulaire, et la supplémentation desdits milieux de culture cellulaire avec un polymère greffé dans lequel des groupements N-vinylcaprolactame et acétate de vinyle sont greffés sur un squelette de polyéthylène glycol, dans lequel le polymère greffé est un produit de i) 30 à 70 % en poids de fragments N-vinylcaprolactame, ii) 15 à 35 % en poids de groupements acétate de vinyle, et iii) 10 à 30 % en poids d'un polyéthylène glycol, à condition que le total de i), ii) et iii) soit égal à 100 % en poids, le polymère greffé étant présent en des quantités de 0,05 à 5 % en poids, sur la base de la quantité totale du milieu de culture cellulaire.

2. Procédé selon la revendication 1, dans lequel le polymère greffé est un produit de i) 40 à 60 % en poids de groupements N-vinylcaprolactame, ii) 15 à 35 % en poids de groupements acétate de vinyle, et iii) 10 à 30 % en poids d'un polyéthylène glycol.

3. Procédé selon la revendication 1 ou 2, dans lequel le polymère greffé PEG-VCap-VAc est un produit de 57 % en poids de N-vinylcaprolactame, 30 % en poids d'acétate de vinyle et 13 % en poids de polyéthylène glycol PEG 6000.

4. Procédé selon l'une quelconque des revendications 1 à 3, dans lequel le polymère greffé est présent en des quantités de 0,5 à 3 % en poids, par rapport à la quantité totale du milieu.

5. Procédé selon l'une quelconque des revendications 1 à 4, dans lequel le polymère greffé est utilisé en combinaison avec un second stabilisant.

6. Procédé selon la revendication 5, dans lequel le second stabilisant est un protecteur contre le cisaillement.

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060840552 A, Chalmers J
- US 6448371 B
- WO 2015003773 A1
- WO 03084479 A2
- WO 2007051743 A
- WO 201415190 A

**Non-patent literature cited in the description**

- **THARMALINGAM T et al.** *Mol Biotechnol.*, 2008
- **MURHAMMER, D.W. et al.** Sparged animal cell bioreactors: Mechanism of cell damage and Pluronic F- 68 protection. *Biotechnology Progress*, 1990, vol. 6, 391-397
- **MURHAMMER, D.W.** ; **GOOCHEE, C.F.** Structural features of nonionic polyglycol polymer molecules responsible for the protective effect in sparged animal cell bioreactors. *Biotechnology Progress*, 1990, vol. 6, 142-148
- **THARMALINGAM, T. et al.** Pluronic enhances the robustness and reduces the cell attachment of mammalian cells. *Molecular Biotechnology*, 2008, vol. 39, 167-177
- Small-scale liquid fermentations. **HILTON, M.D.** Manual of Industrial Microbiology and Biotechnology. American Society of Microbiology, 1999
- **PENG H et al.** *Biotechnol. Progress*, 2014
- **HU W et al.** *Biotechnology and Bioengineering*, 2008
- **HU, W.** *Biotechnology and Bioengineering*, 01 September 2008, vol. 101 (1)
- **WUU, J. et al.** Evaluating sugar-based detergents as a potential alternative to poloxamer bubble protectant. *Conference on Cell Culture Engineering*, vol. XV
- **GRIMSUD, P.A. et al.** Oxidative Stress and Covalent Modification of Protein with Bioactive Aldehydes.. *Journal of Biological Chemistry*, vol. 32 (283), 21837-21841
- **MICHAEL LINN et al.** Soluplus as an effective absorption enhancer of poorly soluble drugs in vitro and in vivo. *EUROPEAN JOURNAL OF PHARMACEUTICAL SCIENCES*, 14 February 2012, vol. 45 (3), 336-343
- **HAOFAN PENG et al.** Mechanism investigation for poloxamer 188 raw material variation in cell culture. *BIOTECHNOLOGY PROGRESS*, 01 May 2016, vol. 32 (3), 767-775
- **SAKAI KENTARO et al.** Use of nonionic surfactants for effective supply of phosphatidic acid in serum-free culture of Chinese hamster ovary cells. *JOURNAL OF BIOSCIENCE AND BIOENGINEERING*, 01 January 2001, vol. 92 (3), 256-261
- **HOSSLER, P.** Improvement of Mammalian Cell Culture Performance Through Surfactant Enabled Concentrated Feed Media. *Biotechnol.Prog.*, 2013, vol. 29 (4), 1023-1032
- **REINTJES, T.** *BASF Pharma Polymers Solubilizer Compendium, Lampertheim*, 2011
- **GRONEMEYER, P. et al.** Trends in Upstream and Downstream Process Development for Monoclonal Antibodies. *Bioengineering*, 2014, vol. 1, 188-212